(19) Europäisches Patentamt
European Patent Office
Office européen des brevets

(11) Veröffentlichungsnummer: **0 076 957**
B1

(12) # EUROPÄISCHE PATENTSCHRIFT

(45) Veröffentlichungstag der Patentschrift: 09.04.86

(21) Anmeldenummer: 82108882.0

(22) Anmeldetag: 25.09.82

(51) Int. Cl.⁴: **C 07 C 103/16**, C 07 C 103/26, C 07 C 103/19, C 07 C 131/00, C 07 D 277/32, A 01 N 37/36, A 01 N 37/44, A 01 N 39/02, A 01 N 43/00

(54) **Substituierte Hydroxymalonsäurediamide, Verfahren zu ihrer Herstellung sowie ihre Verwendung als Schädlingsbekämpfungsmittel.**

(30) Priorität: **10.10.81 DE 3140275**

(43) Veröffentlichungstag der Anmeldung:
**20.04.83 Patentblatt 83/16**

(45) Bekanntmachung des Hinweises auf die Patenterteilung:
**09.04.86 Patentblatt 86/15**

(84) Benannte Vertragsstaaten:
**AT BE CH DE FR GB IT LI NL SE**

(56) Entgegenhaltungen:
**EP - A - 0 001 450**
**US - A - 3 946 074**

**CHEMICAL ABSTRACTS, Band 82, Nr. 1, 6. Januar 1975, Seite 179, Nr. 1919m, Columbus, Ohio, USA "Armillariella tabescens. II. Isolation and the structure of armillarisins"**
**JOURNAL OF THE AMERICAN CHEMICAL SOCIETY, Band 74, Nr. 4, 27. Februar 1957, Seiten 894-895 R.H. PATTON et al.: "The acid hydrolysis of fluorocarbon carboxylic acid caynide dimers and the products thereof"**
**CHEMICAL ABSTRACTS, Band 59, Nr. 8, 14. Oktober 1963, Spalte 8610a, Columbus, Ohio, USA, OSMAN ACHMATOWICZ et al.: "Monoenic syntheses. I. On monoenophilic reactivity of ethyl mesoxalate"**

(73) Patentinhaber: **BAYER AG, Konzernverwaltung RP Patentabteilung, D-5090 Leverkusen 1 Bayerwerk (DE)**

(72) Erfinder: **Findeisen, Kurt, Dr., In der Follmuehle 10, D-5068 Odenthal 2 (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

EP 0 076 957 B1

**Beschreibung**

Die vorliegende Erfindung betrifft neue substituierte Hydroxy-malonsäurediamide, ein Verfahren zu Ihrer Herstellung sowie ihre Verwendung als Schädlingsbekämpfungsmittel.

Es ist bereits bekannt geworden, daß Carbamate wie 5,6-Dimethyl-2-dimethylamino-4-pyrimidinyl-dimethylcarbamat oder 1-Naphthyl-N-methylcarbamat insektizide Wirksamkeit besitzen (US-PS 3 493 574, 2 903 478). Ihre Wirkung ist jedoch vor allem bei niedrigen Aufwandkonzentrationen nicht immer voll befriedigend.

Es wurden die neuen substituierten Hydroxy-malonsäurediamide der Formel (I) gefunden

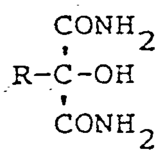

(I)

in welcher

R für Alkyl mit mindestens 2 C-Atomen, sowie für durch Chlor substituiertes Alkyl, gegebenenfalls substituiertes Alkenyl, gegebenenfalls substituiertes Cycloalkyl, substituiertes Aryl, gegebenenfalls substituiertes Heteroaryl steht.

Weiter wurde gefunden, daß man die substituierten Hydroxymalonsäurediamide der Formel I

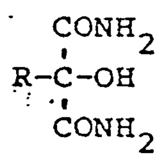

(I)

in welcher

R für Alkyl mit mindestens 2 C-Atomen sowie für durch Chlor substituiertes Alkyl, gegebenenfalls substituiertes Alkenyl, gegebenenfalls substituiertes Cycloalkyl, substituiertes Aryl, gegebenenfalls substituiertes Heteroaryl steht,

erhält

a) wenn man die substituierten Trimethylsilyloxymalonsäuredinitrile der Formel (II)

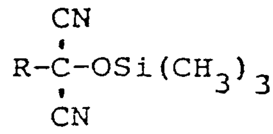

(II)

in welcher

R die oben angegebene Bedeutung hat,

mit anorganischen Säuren verseift, oder

b) Hydroxymalonsäureesteramide der Formel III

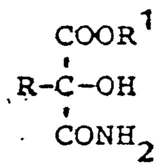

(III)

in welcher

R die oben angegebene Bedeutung hat und

$R^1$ für gegebenenfalls substituiertes Alkyl oder gegebenenfalls substituiertes Cycloalkyl steht

mit Ammoniak umsetzt

oder

c) Hydroxymalonsäureester der Formel IV

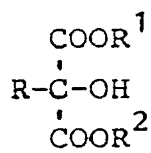

(IV)

in welcher

R bzw. $R^1$ die oben angegebene Bedeutung haben und

$R^2$ die für $R^1$ angegebene Bedeutung hat, wobei $R^1$ und $R^2$ gleich oder verschieden sein können

mit Ammoniak umsetzt

oder

d) Hydroxy- bzw. Trimethylsilyloxymalonsäureamidnitrile der allgemeinen Formel V

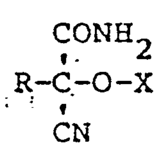

(V)

in welcher

R die oben angegebene Bedeutung hat und

X für Wasserstoff oder $Si(CH_3)_3$ steht

mit anorganischen Säuren verseift

oder

e) Acyloxymalonsäurediamide der allgemeinen Formel

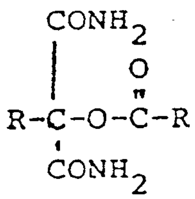

(VI)

in welcher

R die oben angegebene Bedeutung hat

in Gegenwart von Basen verseift

oder

f) Acyloxymalonsäuredinitrile der allgemeinen Formel VII

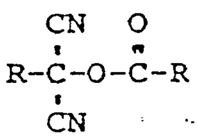

(VII)

in welcher

R die oben angegebene Bedeutung hat

mit anorganischen Mineralsäuren verseift

oder

g) Acyloxymalonsäurediester der allgemeinen Formel VIII

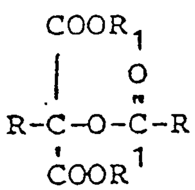

(VIII)

in welcher

R und $R^1$ die oben angegebene Bedeutung haben

mit Ammoniak umsetzt.

Überraschenderweise zeigen die erfindungsgemäßen substituierten Hydroxymalonsäurediamide eine erheblich höhere insektizide Wirkung als die aus dem Stand der Technik bekannten Carbamate der gleichen Wirkungsrichtung. Die erfindungsgemäßen Stoffe eignen sich somit zur Schädlingsbekämpfung.

Weiter wurde gefunden, dass substituierte Hydroxymalonsäurediamide der Formel Ia

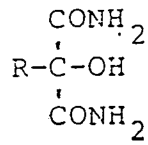

(Ia)

in welcher

R für Alkyl mit mindestens 2 C-Atomen sowie für substituiertes Alkyl gegebenenfalls substituiertes Alkenyl gegebenenfalls substituiertes Cycloalkyl, gegebenenfalls substituiertes Aryl, gegebenenfalls substituiertes Heteroaryl steht,

zur Bekämpfung von Schädlingen verwendet werden können.

Bevorzugt sind Verbindungen der Formel I, in welcher

R für $C_{2-4}$-Alkyl, für durch Chlor substituiertes $C_{1-4}$-Alkyl, für $C_{2-7}$-Alkenyl, $C_{3-6}$-Cycloalkyl steht, die gleich oder verschieden durch einen oder mehrere der folgenden Reste substituiert sind:

Halogen, $C_{1-4}$-Alkoxy, Carboxyl, Carbalkoxy, Phenyl, Phenoxy, Thiophenyl wobei die Phenylringe durch Halogen oder Alkyl substituiert sein können;

oder in welcher R ferner für Phenyl steht, das gleich oder verschieden durch einen oder mehrere der folgenden Reste substituiert ist: Halogen, insbesondere Chlor, Brom, Fluor, Nitro, Amino, CN, OH, $C_{1-4}$-Alkyl insbesondere Methyl, $C_{1-4}$-Halogenalkyl insbesondere Trifluormethyl, Trichlormethyl, Pentafluorethyl, $C_{1-4}$-Alkoxy, $C_{1-4}$-Halogenalkoxy, insbesondere Trifluormethoxy, Pentafluorethoxy, Methylendioxy, Ethylendioxy, Difluormethylendioxy, halogensubstituiertes Ethylendioxy, $C_{1-4}$-Alkylthio, $C_{1-4}$-Halogenalkylthio insbesondere Trifluormethylthio, $C_{2-8}$-Alkoxyalkyl, $C_{2-8}$-Halogenalkoxyalkyl, $C_{1-4}$-Alkyl-sulfonyl insbesondere Methylsulfonyl, $C_{1-4}$-Halogenalkylsulfonyl, Carboxyl, Carbalkoxy insbesondere Methoxycarbonyl, sowie den

Rest $C_{1-4}$-Alkoxy-N=CH-, insbesondere $CH_3$-O-N=CHferner für Phenyl, Phenyloxy, Thiophenyl die gegebenenfalls durch Halogen oder $C_{1-4}$-Alkyl substituiert sein können, sowie für Carbalkoxy mit 2 - 4 C-Atomen, wie Carboxymethoxy,

oder wobei R ferner für Pyridinyl, Pyrimidinyl, Triazinyl, Isoxyzolyl, Thiazolyl, Oxadiazolyl, Imidazolyl, Triazolyl, Furanyl, Thiophenyl steht, die gegebenenfalls ein oder mehrfach gleich oder verschieden durch Halogen insbesondere Chlor, $C_{1-4}$-Alkyl, insbesondere Methyl oder Ethyl, $C_{1-4}$-Alkoxy, insbesondere Methoxy, Ethoxy substituiert sein können.

Besonders bevorzugt sind Verbindungen der Formel I in welcher

R für $C_{2-4}$-Alkyl für chlorsubstituiertes $C_{1-4}$-Alkyl sowie für durch Halogen, Phenyl oder Phenoxy substituiertes $C_{5-6}$-Cycloalkyl, für gegebenenfalls durch Carboxyl substituiertes $C_{2-4}$-Alkenyl ferner für Phenyl, das durch Halogen, insbesondere

Fluor oder Chlor, $C_{1-4}$-Alkyl, $C_{1-4}$-Alkoxy, $C_{1-4}$-Halogenalkoxy, $C_{1-4}$-Halogenalkyl, $NH_2$, $CH_3O$-N = CH-, oder Nitro substituiert ist, steht.

Ganz besonders bevorzugt sind Verbindungen der Formel I,

in welcher

R für Phenyl steht, das gegebenenfalls ein- oder mehrfach durch Chlor oder Fluor substituiert ist.

Als neue Wirkstoffe seien im einzelnen genannt:

o-, m-, p-Chlorphenyl-hydroxy-malonsäurediamid, 2,3-Dichlorphenylhydroxy-malonsäurediamid, 3,4-Dichlor phenyl-hydroxymalonsäurediamid, 3,5-Dichlor phenyl-hydroxy-malonsäurediamid, 2,4-Dichlor phenyl-hydroxy-malonsäurediamid, 2,5-Dichlorphenyl-hydroxy-malonsäurediamid, 2,6-Dichlor-phenyl-hydroxy-malonsäurediamid, o-, m-, p-Nitrophenylhydroxy-malonsäurediamid, o-Chlor -methylphenyl-hydroxymalonsäurediamid, o-, m-, p-Trifluormethylphenyl-hydroxy-malonsäurediamid, o-, m-, p-Methoxyphenyl-hydroxymalonsäurediamid, 2,6-Dimethoxyphenyl-hydroxy-malonsäurediamid, o-, m-, p-Tolylhydroxy-malonsäurediamid,

o-, m-, p Trifluormethoxyphenyl-hydroxy-malonsäurediamide, o-, m-, p-Fluorphenylhydroxymalonsäurediamide,

Cyclohexyl-hydroxy-malonsäurediamid,

Butyl-hydroxy-malonsäurediamid, tert.-Butyl-hydroxy-malonsäurediamid, Fluor -tert.-butyl-hydroxy-malonsäurediamid, Chlor -tert.-butyl-hydroxy-malonsäurediamid, Difluor -tert.-butyl-hydroxy-malonsäurediamid, Trichlor methylhydroxy-malonsäurediamid.

Verwendet man bei Verfahren a) Trimethylsilyloxy- p-chlorphenylmalonsäuredinitril als Ausgangsstoff und 96%ige Schwefelsäure als Verseifungsmittel, so kann der Reaktionsablauf durch das folgende Formelschema wiedergegeben werden:

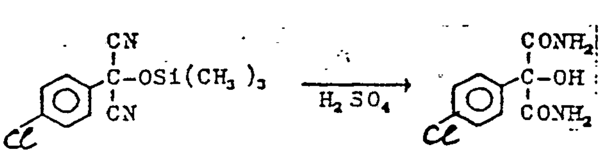


Die als Ausgangsprodukte verwendeten Trimethyl-silyloxymalonsäuredinitrile der Formel II sind zum Teil bekannt (Chem. Ber. 106, 587 (1973), Tetrahedron Letters No. 17, 1449 - 1450 (1973)). Neue Verbindungen können nach den dort angegebenen Methoden leicht entsprechend dem folgenden Formelschema syntetisiert werden.

$$R\text{-}COCl + 2\ (CH_3)_3SiCN \longrightarrow$$

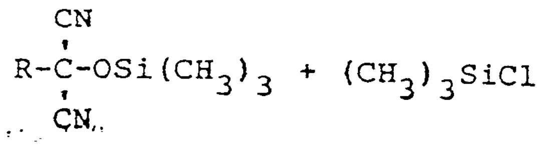

wobei R die vorgenannte Bedeutung besitz.

In der Formel II steht R vorzugsweise für geradkettige oder verzweigte Alkylreste mit 2 - 6 Kohlenstoffatomen. Ferner für $C_{1-6}$-Alkyl das durch Chlor substituiert ist, weiterhin vorzugsweise für Alkenylreste mit 2 bis 7 Kohlenstoffatomen, die durch Halogen wie Fluor, Chlor, Brom, Alkoxy, Carboxyl oder Carbalkoxygruppen substituiert sein können, weiterhin vorzugsweise für Cycloalkylreste mit 3 bis 6 Kohlenstoffatomen, die durch Halogen wie Fluor, Chlor, Brom, Alkoxy, Carbalkoxygruppen substituiert sein können, R steht weiterhin vorzugsweise für Aryl insbesondere Phenyl, das durch Alkyl mit 1-4 Kohlenstoffatomen, substituiertes Alkyl, das durch Halogenwie Fluor oder Chlor und Alkoxygruppen substituiert ist, Aryl das weiterhin durch Halogen, wie Fluor, Chlor, Brom, $C_{1-4}$-Alkoxy-, durch Chlor oder Fluor substituiertes $C_{1-4}$-Alkoxy, $NH_2$ oder Nitrogruppen substituiert ist, R steht weiterhin vorzugsweise für Heteroaryl, das durch Alkyl mit 1 bis 4 Kohlenstoffatomen, Alkoxygruppen oder Halogen wir Fluor, Chlor und Brom substituiert sein kann.

Als Verdünnungsmittel bei der Verseifung gemäß Verfahren a) verwendet mam im allgemeinen Wasser. Die saure Verseifung kann in wasserfreien- oder Wasser enthaltenden Säuren durchgeführt werden, häufig genügen schon geringe Mengen Wasser, um die gewünschten Endprodukte zu erhalten.

Die Wassermenge kann aber in einem größeren Bereich, zwischen 2 % und 75 % bezogen auf die eingesetzte Säuremenge, variiert werden. Es ist weiterhin möglich, die Ausgangsprodukte der Formel II zuerst in einer wasserfreien Säure zu lösen und die benötigte Wassermenge zu einem späteren Zeitpunkt hinzuzufügen.

Als geeignete Säuren seien vorzugsweise genannt: Schwefelsäure, Phosphorsäure, Salzsäure, Flußsäure, Borsäure. Besonders bevorzugt sind Schwefelsäure ($H_2SO_4$) und Salzsäure (HCl).

In einigen Fällen sind die Endprodukte der allgemeinen Formel I etwas wasserlöslich und müssen deshalb mit Hilfe eines Extraktionsmittels aus diesem entfernt werden. Als Extraktionsmittel kommen alle inerten organischen Lösungsmittel, die sich mit Wasser nicht, oder nur wenig, mischen, in Betracht. Hierzu gehören Toluol, Xylol, Chlorbenzol, Dichlorobenzol, Essigsäureethylester, Methylenchlorid, Chloroform, Tetrachlorkohlenstoff und Ether. Im allgemeinen ist ein Extraktionsmittel aber nicht nötig.

Die Reaktionstemperaturen können in einem

größeren Bereich variiert werden. Im allgemeinen arbeitet man zwischen etwa −15°C bis 100°C, vorzugsweise zwischen 0 und 80°C, insbesondere zwischen 20 und 60°C.

Die Umsetzung wird normalerweise bei Normaldruck. Sie kann aber auch bei erhöhtem Druck durchgeführt werden. Im allgemeinen arbeitet man bei Drücken zwischen etwa 1 bar und etwa 10 bar, vorzugsweise zwischen 1 bar und 5 bar.

Bei der Durchführung des erfindungsgemäßen Verfahrens a) setzt man auf 1 Mol der substituierten Trimethylsilyloxymalonsäuredinitrile (Formel II) etwa 0,5 bis 20 Mol, vorzugsweise 1 bis 5 Mol an anorganischer Säure ein.

Die in der Säure gelöste Verbindungen der Formel II wird nach 30 Minuten bis zwei Stunden Rühren auf Eis gegossen und entweder durch Absaugen oder Extraktion gewonnen. Die Reinigung wird im allgemeinen durch Umkristallisation vorgenommen.

Verfahren b wird durchgeführt indem man Hydroxymalonsäureesteramide (III) mit Ammoniak nach folgendem Formelschema umsetzt:

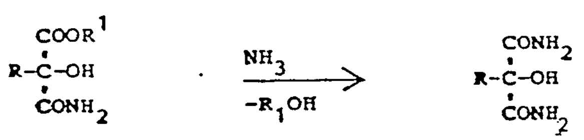


III

Bevorzugt sind Verbindungen der Formel III in welcher R für die weiter oben angegebene bevorzugten Definitionen steht und $R^1$ für $C_{1-4}$-Alkyl, das gegebenenfalls durch Halogen, insbesondere Chlor, oder $C_{1-4}$-Alkoxy substituiert ist, steht. Insbesondere steht $R^1$ für Methyl, Ethyl oder Propyl.

Im allgemeinen wird in gegenüber den Edukten und Produkten inerten Lösungsmitteln gearbeitet.

Bevorzugte Lösungsmittel sind Alkohole wie Methanol, Ethanol, Propanol, Isopropanol, Kohlenwasserstoffe wie Toluol, Xylol, Halogenkohlenwasserstoffe wie Chlorbenzol, Methylenchlorid, Tetrachlorkohlenstoff und Ether wie Diethylether, Dioxan. Selbstverständlich sind auch Mischungen der Lösungsmittel geeignet. Besonders bevorzugt sind die Alkohole.

Auf 100 ml Lösungsmittel werden 5 - 100 g, bevorzugt 10 - 70 g des Hydroxymalonsäureesteramids eingesetzt.

Die Reaktion wird bei Temperaturen von −0°C - 100°C, bevorzugt bei Temperaturen von 0 - 80°C, besonders bevorzugt bei Temperaturen von 20 - 60°C durchgeführt.

Im allgemeinen wird in die Lösung der Ausgangsverbindung der Formel III Ammoniak gasförmig eingeleitet, wobei mindestens die der Stöchiometrie entsprechende Menge Ammoniak eingesetzt werden muß.

Bevorzugt wird aber mit einem Überschuß an Ammoniak gearbeitet. Unter Umständen muß auch unter Druck gearbeitet werden.

Es ist aber ebenso möglich eine Lösung des Ausgangsproduktes mit einer gegebenenfalls wässrigen $NH_3$-Lösung zu mischen. Es kann auch in üblicher Weise im Zweiphasen-System gearbeitet werden.

Die Reaktion verläuft schnell und deshalb kann fast sofort nach Beendigung der Ammoniakeinleitung aufgearbeitet werden.

Sofern nicht das schwer lösliche Endprodukt schon während der Reaktion oder durch Abkühlen ausfällt und abgesaugt werden kann wird eingeengt und das fast reine Produkt der Formel I - gegebenenfalls durch Umkristallisation von Spuren Verunreinigung befreit - erhalten.

Die Verbindungen der Formel III sind neu.

Sie werden erhalten, indem man Hydroxy- bzw. Silyloxymalonesternitrile der allgemeinen Formel (IX) mit anorganischen Säuren nach folgendem Formelschema verseift:

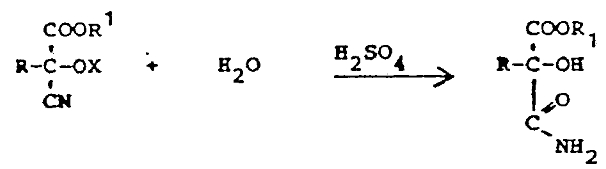


IX III

Bevorzugte Verbindungen der Formel IX sind diejenigen in denen die Reste R und $R^1$ die weiter oben angegebene bevorzugte Definition besitzen und X für Wasserstoff steht.

Für die Durchführung der Verseifung gilt das für die Verseifung der Verbindungen der Formel II gesagte (vgl. Verfahren a)).

Es ist vorteilhaft bei der Aufarbeitung bei Temperaturen unter 10°C zu arbeiten um eine eventuelle Hydrolyse der Estergruppe im sauerwässrigen-Milieu auszuschließen.

Die Verbindungen der Formel IX sind neu.

Sie werden erhalten indem man α-Ketocarbonsäureester der allgemeinen Formel X

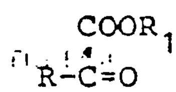

X

wobei

R und $R_1$ die oben angegebene Bedeutung haben

mit HCN oder HCN abspaltenden Verbindungen oder mit Trimethylsilylcyanid umsetzt.

Dies sei durch folgende 3 Reaktionsgleichungen illustriert.

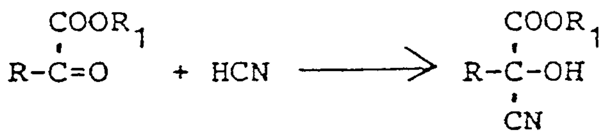

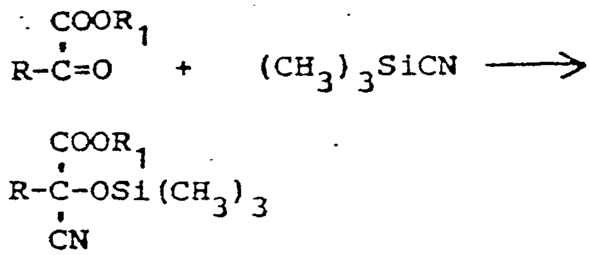

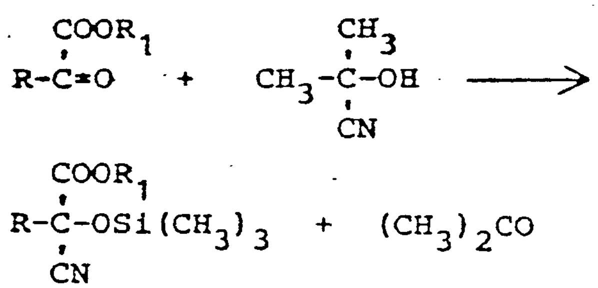

Als HCN-abspaltende Substanzen kommen hierfür Cyanhydrine zum Einsatz, beispielsweise seien Acetoncyanhydrin oder Benzaldehydcyanhydrin genannt.

Die Umsetzung von X zu IX verläuft nach den in der Literatur bekannten Methoden unter Anwesenheit geringer Mengen alkalischer Katalysatoren, beispielsweise Natriumcyanid, Kaliumcyanid oder tertiäre Amine wie Triethylamin sowie Alkali- und Erdalkalihydroxide und -carbonate.

Während die Addition von molaren Mengen HCN bzw. Trimethylsilylcyanid leicht exotherm bereits bei Raumtemperatur verläuft und nach beendeter Zugabe der Reagenzien abgeschlossen ist muß bei Einsatz von Cyanhydrinen einige Zeit erhitzt werden um einen vollständigen Umsatz zu erreichen. Hierbei ist es auch sinnvoll auf 1 Mol α-Ketoester der Formel X mehr als 1 Mol Canhydrin anzusetzen.

Bevorzugt werden 1,1 - 3 Mol, ganz besonders bevorzugt 1,2 - 2 Mol Cyanhydrin verwendet. Der Überschuß wird nach beundeter Reaktion wieder abdestilliert.

Die Reaktionen verlaufen im allgemeinen ohne Anwesenheit von Lösungsmitteln, jedoch kann die Verwendung von gegenüber den Produkten und Edukten inerten Lösungsmitteln wie z.B. Methanol, Ethanol, Isopropanol, Toluol, Chlorbenzol, Methylenchlorid, Tetrachlorkohlenstoff und Diethylether in einigen Fällen durchaus sinnvoll sein.

Die Reaktionsbedingungen für die Umsetzungen sind sehr variabel. So können z.B. auch HCN und Trimethylsilylcyanid überstöchiometrisch eingesetzt werden, jedoch bringt ein mehr als 10 %iger molarer Überschuß keinerlei Vorteile mehr. Ebenso ist die Reaktionstemperatur in weiten Grenzen variierbar, es kann im Temperaturbereich von – 50 bis 300° C gearbeitet werden, wobei bei höheren Temperaturen entweder in der Gasphase oder in der Flüssigphase unter Druck die Umsetzungen vorgenommen werden. Aber auch hier ergibt sich gegenüber den zuerst beschriebenen bevorzugten Verfahrensbedingungen kein signifikanter Vorteil.

Verbindungen der Formel X sind bekannt bzw. lassen sich analog zu bekannten Methoden darstellen (S. Hünig, R. Schaller, Angewandte Chemie 94, 10 (1982)).

Verfahren c wird durchgeführt indem man Hydroxymalonsäurediester der Formel IV mit Ammoniak nach folgendem Formelschema umsetzt:

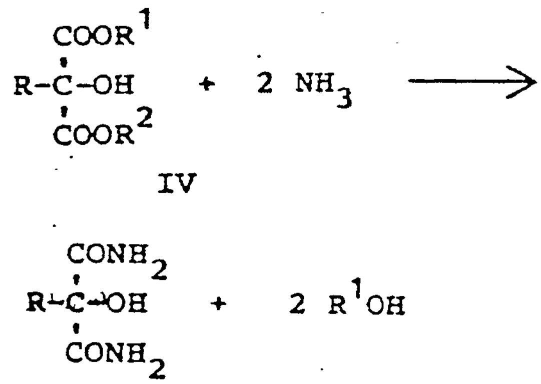

IV

Für die Durchführung der Amidierung gilt das für die Umsetzung der Verbindungen der Formel III (vgl. Verfahren b) gesagte.

Die Verbindungen der Formel IV sind teilweise bekannt (Chimica e. Industria 1964, 5, 509 - 517).

Bevorzugte Verbindungen der Formel IV sind diejenigen in denen die Reste R und $R^1$ die oben angegebenen bevorzugten Bedeutungen besitzen. $R^2$ besitzt dieselben bevorzugten Bedeutungen wie in die oben für $R^1$ angegeben worden sind. $R^1$ und $R^2$ können gleich oder verschieden sein.

Sie werden erhalten indem man entweder gemäß

C1) Verbindungen der Formel IX

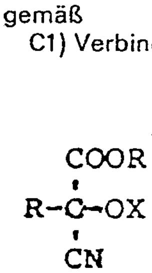

IX

in welcher

R und $R^1$ die oben angegebene Bedeutung haben, mit einem Alkohol der Formel

$R^2$ - OH

in welcher

$R^2$ die oben angegebene Bedeutung hat verestert oder

C2) Verbindungen der Formel II

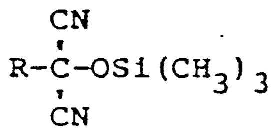

(II)

verestert.

Verfahren C1 zur Herstellung der Verbindungen der Formel IV wird durchgeführt indem man nach der Methode von Pinner (A. Pinner, die Imidoether und ihre Derivate, Berlin 1882) arbeitet.

Hierbei wird im allgemeinen die nitrilgruppenhaltige Verbindung entweder mit Salzsäure oder mit Schwefelsäure in alkoholischer Lösung ($R_2OH$) und Wasser umgesetzt.

Im folgenden werden beide Methoden beispielhaft beschrieben, ohne jedoch deshalb die Allgemeingültigkeit der Verfahren einzuschränken.

a.) Verseifung mit Salzsäure:

Hierbei wird die Verbindung der Formel IX mit 2 20 molarem Überschuß an absolutem Alkohol, der-Formel $R^2OH$ bei $-10$ bis 5°C mit 1,1 - 20 Mol HCl-Gas behandelt. Dann wird ca. 1 Stunde bei unter 30°C nachgerührt und anschließend mit der stöchiometrischen Menge $H_20$ versetzt und ca. eine weitere Stunde bei 0 - 50°C gerührt. Anschließend wird abgesaugt und das Filtrat eingeengt.

Der Rückstand wird mit einem inerten Lösungsmittel z.B. Methylenchlorid aufgenommen, mit Wasser bei 0 - 10°C gewaschen und nach Entfernen des Lösungsmittels gegebenenfalls durch Destillation gereinigt.

Die Reaktion kann aber auch invers vorgenommen werden, d.h. die Verbindung der Formel IX wird zwischen $-20$ und $+10$°C in eine Alkohol-Salzsäure-Lösung eingebracht.

b.) Verseifung mit Schwefelsäure:

Auf 1 Mol der Verbindung der Formel IX werden 100-1000 g Schwefelsäuremonohydrat, 2 - 25 Mol des Alkohols der Formel $R^2OH$ sowie 1 Mol Wasser eingesetzt. Ein stärkeres Abweichen von der Stöchiometrie bei der Dosierung der Wassermenge führt zu Ausbeuteverlusten. Ein eventueller Wassergehalt der Ausgangsprodukte (Alkohol und Schwefelsäure) muß bei der Menge zuzusetzenden Wasser berücksichtigt werden. Die Ansatzlösung wird bei Temperaturen von 0 - 150°C, bevorzugt 20 - 100°C, besonders bevorzugt 60 - 90°C, 1 - 20 Stunden gerührt. Gegebenenfalls kann unter erhöhtem Druck gearbeitet werden. Dann wird in Eiswasser eingerührt und mit einem gebräuchlichen organischen Lösungsmittel wie z.B. Methylenchlorid, Toluol, Essigsäureethylester kalt extrahiert und das Produkt gegebenenfalls durch Destillation gereinigt.

Verfahren C2 zur Herstellung der Verbindungen der Formel IV wird nach der bereits beschriebenen Methoden von Pinner (vgl. Verfahren C1) durchgeführt, wobei im Falle des Arbeitens mit Salzsäure allerdings nach der inversen Methode gearbeitet werden muß, da ein Vorlegen der Verbindungen der Formel II im Alkohol nicht möglich ist.

In diesem Fall käme es zur Zerstörung der Verbindungen der Formel II.

Im folgenden soll beispielhaft die Reaktionsdurchführung für die inverse Pinner-Veresterung beschrieben werden, ohne daß damit die allgemeine Durchführbarkeit der Reaktion eingeschränkt wird:

Der absolute Alkohol der Formel $R^2OH$ (wobei $R^2$ die weiter oben angegebene Bedeutung hat) wird im 2 - 20 fachen molaren Überschuß - bezogen auf die Verbindung der Formel II - vorgelegt und bei $-10$ bis $+10$°C mit HCl-Gas gesättigt. Hierbei müssen für 1 Mol Verbindung der Formel II mindestens 2 Mol HCl aufgenommen werden. Bei $-20$ bis $+10$°C, bevorzugt bei $-10$°C wird die Reaktionskomponente der Formel II zugetropft und anschließend ca. 1 Stunde bei max. 30°C nachgerührt, bevor mit der stöchiometrischen Menge Wasser - bezogen auf eingesetzte CN-Gruppen, gegebenenfalls verdünnt im Alkohol, versetzt wird.

Ein zu starker Über- oder Unterschuß der Wassermenge führt zu Ausbeuteverlusten.

Nachdem noch ca. 1 Stunde bei 0 - 50°C nachgerührt worden ist, wird analog zu Verfahren C1 aufgearbeitet.

Wird das Verfahren C2 in Schwefelsäure durchgeführt, so wird analog zu der bei Verfahren C1 beschriebenen Schwefelsäurevariante gearbeitet. Besonders bevorzugt wird hierbei aber die Verfahrensvariante, bei der Alkohol, Säure und Wasser vorgelegt werden und sodann die Verbindungen der Formel II zugegeben wird.

Von einer Destillation der Verbindungen der Formel IV sollte abgesehen werden, wenn deren Siedepunkte bei Destillationsbedingungen 160°C deutlich überschreiten.

Verfahren d) wird durchgeführt indem man Hydroxy- bzw. Trimethylsilyloxymalonsäureamidnitrile der allgemeinen Formel V mit anorganischen Säuren nach folgendem Formelschema verseift:

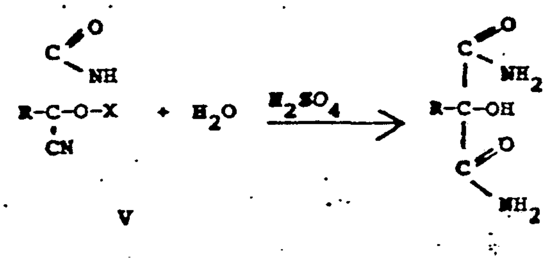


V

Für die Durchführung der Verseifung gilt das für die Verseifung der Verbindung der Formel II gesagte (vgl. Verfahren a)).

Verbindungen der Formel V sind neu.

Bevorzugt sind Verbindungen der Formel V wobei R die weiter oben angegebene bevorzugte Definition besitzt

Sie werden erhalten, indem man α-Ketosäureamide der allgemeinen Formel XI

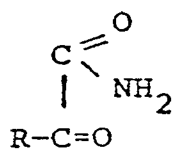

XI

wobei

R die oben angegebene Bedeutung hat mit HCN oder HCN abspaltenden Mitteln oder mit Trimethylsilylcyanid umsetzt.

Dies sei durch folgende 3 Reaktionsgleichungen illustriert:

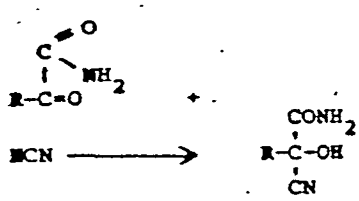

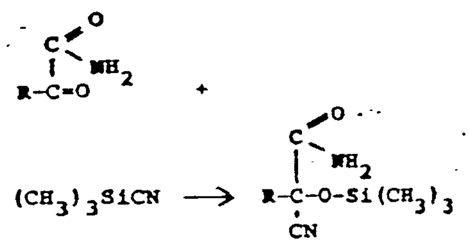

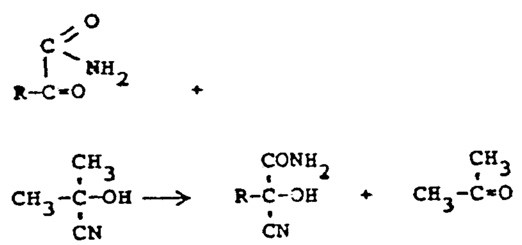

Als HCN-abspaltende Substanzen kommen hierfür Cyanhydrine zum Einsatz, beispielsweise seien Acetoncyanhydrin oder Benzaldehydcyanhydrin genannt.

Die Umsetzung von XI zu V verläuft nach der in der Literatur bekannten Methoden unter Anwesenheit geringer Mengen alkalischer Katalysatoren, beispielsweise Natriumcyanid, Kaliumcyanid oder tertiäre Amine wie Triethylamin, sowie Alkali- und Erdalkalihydroxide und -carbonate.

Während die Addition von molaren Mengen HCN bzw. Trimethylsilylcyanid leicht exothern bereits bei Raumtemperatur verläuft und nach beendeter Zugabe der Reaganzien abgeschlossen ist muß bei Einsatz von Cyanhydrinen einige Zeit erhitzt werden um einen vollständigen Umsatz zu erreichen. Hierbei ist es auch sinnvoll auf 1 Mol α-Ketoamid der Formel XI mehr als 1 Mol Cyanhydrin anzusetzen. Bevorzugt werden 1,1 - 3 bevorzugt 1,2 - 2 Mol Cyanhydrin verwendet. Der Überschuß wird nach beendeter Reaktion wieder abdestilliert.

Die Reaktionen verlaufen im allgemeinen ohne Anwesenheit von Lösungsmittel, jedoch kann die Verwendung von gegenüber den Produkten und Edukten inerten Lösungsmitteln wie z.B. Methanol, Ethanol, Isopropanol, Toluol, Chlorbenzol, Methylenchlorid, Tetrachlorkohlenstoff und Diethylether in einigen Fällen durchaus sinnvoll sein.

Die Reaktionsbedingungen für die Umsetzungen sind sehr variabel. So können z.B. HCN und Trimethylsilylcyanid auch überstöchiometrisch eingesetzt werden, jedoch bringt ein mehr als 10 %iger molarer Überschuß keinerlei Vorteile mehr. Ebenso ist die Reaktionstemperatur in weiten Grenzen variierbar, es kann im Temperaturbereich von -50 bis 300°C gearbeitet werden, wobei bei höheren Temperaturen entweder in der Gasphase oder in der Flüssigphase unter Druck die Umsetzungen vorgenommen werden. Aber auch hier ergibt sich gegenüber den zuerst beschriebenen bevorzugten Versuchsparametern kein signifikanter Vorteil.

Verbindungen der Formel XI sind bekannt bzw. lassen sich analog zu bekannten Methoden darstellen (S. Hünig, K. Schaller, Angewandte Chemie 94, 10 (1982)).

Verfahren e wird durchgeführt indem man Acyloxymalonsäurediamide der allgemeinen Formel VI in Gegenwart von Basen verseift. Die Reaktion läßt sich durch folgendes Formelschema darstellen:

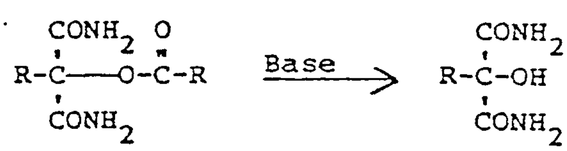


VI

Es werden bevorzugt Verbindungen der Formel VI eingesetzt in welcher R die weiter oben angegebene bevorzugte Bedeutung besitzt.

Die Reaktion wird durchgeführt indem man 1 Mol der Verbindung dei Formel VI in einem inerten Verdünnungsmittel mit in etwa der äquimolaren Menge einer Base versetzt.

Als Verdünnungsmittel dienen Wasser, Alkohole wie Methanol, Ethanol, tert.-Butanol, Kohlenwasserstoffe wie Toluol, Tetrachlorkohlenstoff, Methylenchlorid, Ether wie Diethylether, Dioxan.

Bevorzugt sind Wasser, Methanol oder Ethanol. Es kann auch in üblicher Weise im zweiphasigen System gearbeitet werden.

Als Basen dienen Alkali- und Erdalkalihydroxide, -carbonate, -hydrogencarbonate, -alkoholate wie Natriumhydroxid, Kaliumhydroxid,

Natriumcarbonat, Natriummethylat, Natriumethylat, sowie Ammoniak, Amine z.B. Triethylamin, Pyridin, Dimethylamin.

Die Reaktion wird durchgeführt zwischen -10°C - +60°C bevorzugt zwischen 0 - 30°C.

Die Aufarbeitung nach beendeter Reaktion erfolgt in üblicher Weise.

Die Verbindungen der Formel VI sind teilweise bekannt. Sie können nach bekannten Verfahren hergestellt werden (J. Am. Chem. Soc, 71 S. 34 (1949)).

Verfahren f wird durchgeführt indem man Acyloxymalonsäuredinitrile der Formel VII mit anorganischen Säuren verseift. Die Reaktion läßt sich durch folgendes Formelschema darstellen:

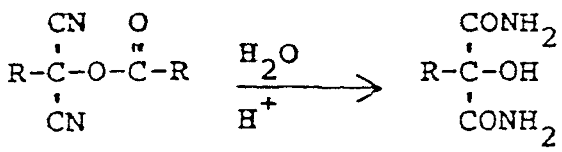

VII

Bevorzugt werden Verbindungen der Formel VII eingesetzt in welcher R die weiter oben angegebene vorzugsweise Bedeutung besitzt.

Verbindungen der Formel VII sind teilweise bekannt. Sie lassen sich nach bekannten Verfahren ßerstellen (vgl. Journal für prakt. Chemie [2] 39, S. 260 (1889), Chemistry a. Industry 1970 S. 1408).

Verfahren f wird duchgeführt indem man die Verbindungen der Formel VII mit konzentrierter Schwefelsäure und 2-10 % Wasser bezogen auf die eingesetzte Schwefelsäure mehrere Stunden bei Temperaturen zwischen 50 und 150°C bevorzugt 60 - 100°C erhitzt.

Verfahren g wird durchgeführt indem man Acyloxymalonsäurediester der Formel VIII mit Ammoniak nach folgendem Reaktionsschema umsetzt:

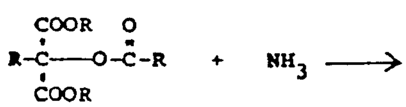

VIII

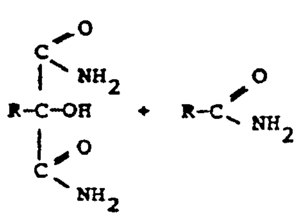

II

Für die Durchführung der Reaktion gilt das für die Amidierung der Hydroxymalonsäurediester der Formel IV nach Verfahren c gesagte. Lediglich die Aufarbeitung wird etwas erschwert durch die Tatsache, daß die bei der Amidierung von Verbindungen der Formel VIII entstehenden beiden Amide infolge ihrer ähnlichen Löslichkeiten etwas schwer durch Kristallisation zu trennen sind.

Verbindungen der Formel VIII sind neu.

Sie werden erhalten, indem man dimere Acylcyanide der Formel VII nach der Methode von Pinner verestert.

Die Bedingungen sind die gleichen wie für die Veresterung der Verbindungen der Formel IX bereits beschrieben (vgl. Verfahren $C_1$ und $C_2$).

Lediglich die Destillation muß auf Grund der hohen Siedepunkte bei einigen Vertretern unterbleiben. In diesen Fall kann aber auch ohne Schwierigkeiten mit dem Rohprodukt weitergearbeitet werden.

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus. Aus der Ordnung der Chilopoda z.B. Geophilus carpophagus, Scutigera spec.

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z.B. Forficula auricularis.

Aus der Ordnung der Isoptera z.B. Reticulitermes spp..

Aus der Ordnung der Anoplura z.B. Phylloxera vastatrix, Pemphigus spp., Pediculus humanus corporis, Haematopinus spp., Linognathus spp.

Aus der Ordnung der Mallophaga z.B. Trichodectes spp., Damalinea spp.

Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae,

Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.. Trogoderma spp., Anthrenus spp., Attagenus spp., Lyctus spp., Meligethes aeneus, Ptinus spp., Niptus hololeucus. Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp. Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp. Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylerylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel

wie Carboxymethylcellulose, natürliche und synthetische pulverige, körnige oder latexförmige Polymere verwendet werden, wie. Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo - und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungegemäßen Wirkstoffe können ferner in ihren handelsüblichen Formulierungen sowie in den aus diesen Formulierungen bereiteten Anwendungsformen in Mischung mit Symergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

**Herstellungsbeispiele:**

**Beispiel 1a**

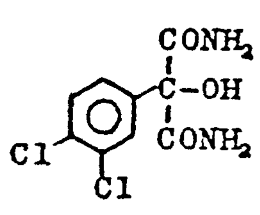

In einem 1 Ltr. Vierhalskolben mit Rückflußkühler, Rührer, Thermometer und Tropftrichter werden 800 g Schwefelsäure (98 %ig) vorgelegt und von Außen gekühlt. Dann tropft man innerhalb von 30 Minuten, wobei die Innentemperatur 50° C nicht übersteigen sollte, 299 g 3,4-Dichlor phenyl-trimethyl-silyloxy-malonsäuredinitril (1 Mol) in die Schwefelsäure. Nachdem die exotherme Reaktion beendet ist, wird 15 Minuten nachgerührt.

Die Reaktionsmischung wird in Eiswasser gegossen, das Reaktionsprodukt fällt aus, wird abgesaugt, neutral gewaschen, getrocknet und der Rückstand aus Ethanol umkristallisiert.

Ausbeute: 242 g 3,4-Dichlor phenyl-hydroxy-malonsäurediamid (≙92 % der Theorie)

Schmelzpunkt: 175 - 177° C.

**Beispiel 1b**

Wie in Beispiel 1 beschrieben, werden 200 g konz. Salzsäure in dem Reaktor vorgelegt und 74,75 g 3,4-Dichlor- phenyl-trimethyl-silyloxy-malonsäuredinitril zugetropft.

Das Reaktionsgemisch wird auf 40° C erwärmt, wobei eine exotherme Reaktion eintritt. Durch Kühlung wird die Innentemperatur bei 40 bis 50° C gehalten. Die Mischung geht in Lösung und fällt gegen Ende der Reaktion teilweise aus. Man gibt auf Eiswasser, saugt das ausgefallende Reaktionsprodukt ab, wäscht mit Eiswasser nach und kristallisiert den Rückstand aus Ethanol um.

Ausbeute: 57,8 g 3,4-Dichlor phenyl-hydroxy-malonsäurediamid (≙ 88 % der Theorie)

Schmelzpunkt: 176 - 177° C.

**Beispiel 2**

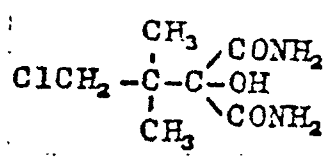

Wie in Beispiel 1 beschrieben, werden 200 g Schwefelsäure (98 %ig) vorgelegt und eine Mischung aus 244 g Chlor- tert.-butyl-trimethylsilyloxy-malonsäuredinitril (1 Mol), gelöst in 150 ml Methylenchlorid bei 0 bis 5°C, zugetropft. Anschließend wird die Kühlung entfernt und die Innentemperatur steigt auf 30°C an. Nach 30 Minuten wird kurz bis zum Rückfluß des Methylenchlorids erwärmt. Das Methylenchlorid wird destillativ entfernt und der Rückstand in 2 Liter Eiswasser eingerührt. Das ausgefallene Produkt wird abgesaugt, mit Wasser neutral gewaschen, getrocknet und aus Ethanol umkristallisiert.

Ausbeute: 181 g Chlor -tert.-butyl-hydroxy-malonsäurediamid (≙ 87 % der Theorie)

Schmelzpunkt: 183 - 185°C.

**Beispiel 3**

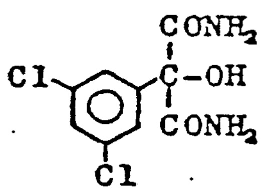

Wie in Beispiel 1 beschrieben, werden 200 g Schwefelsäure (96 %ig) vorgelegt und bei 40 - 50°C unter Kühlung 98 g 3,5-Dichlor phenyl-trimethyl-silyloxy-malonsäuredinitril (0,33 Mol) zugetropft. Anschließend wird eine Stunde bei 60°C nachgerührt. Die Reaktionsmischung wird auf 50-60°C warmes Wasser gegeben, abgesaugt, neutral gewaschen und aus Acetonitril umkristallisiert.

Ausbeute: 75 g 3,5-Dichlor phenyl-hydroxy-malonsäurediamid (≙ 87 % der Theorie)

Schmelzpunkt: 181 - 183°C.

**Beispiel 4**

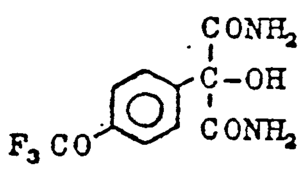

Wie in Beispiel 1 beschrieben, werden 106,5 g 4-Trifluor -methoxyphenyl-trimethylsilyloxy-malonsäuredinitril (0,34 Mol) in 400 g Schwefelsäure (92 %ig) getropft. Anschließend wird 30 Minuten bei 50°C gerührt und dann auf Eis gegeben. Das schmierige Reaktionsprodukt wird in Methylenchlorid aufgenommen, über Natriumsulfat getrocknet, eingeengt und aus Toluol umkristallisiert.

Ausbeute: 79 g 4-Trifluor methoxyphenylhydroxy-malonsäurediamid (≙ 85 % der Theorie)

Schmelzpunkt: 119 - 121°C.

Wie in Beispiel 1 bis 4 beschrieben, wurden die folgenden substituierten Hydroxymalonsäurediamide synthetisiert:

5 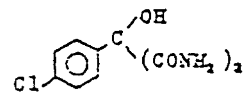 Fp.: 135 - 37°C (aus Chlorbenzol

6 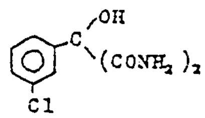 Fp.: 138 - 39°C (aus Xylol)

7 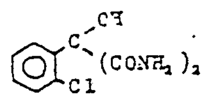 Fp.: 165 - 67°C (aus Ethanol)

8 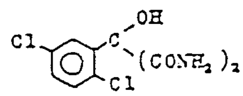 Fp.: 199 - 201°C (aus Ethanol)

9 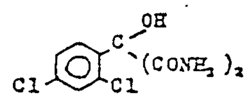 Fp.: 149 - 51°C (aus o-Dichlorbenzol)

10 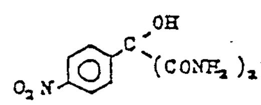 Fp.: 166 - 67°C (aus Ethanol)

11 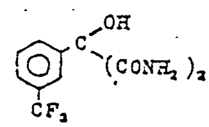 Fp.: 185 - 87°C (aus Ethanol)

12 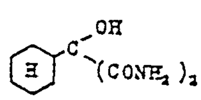 Fp.: 245 - 47°C

13 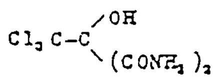 Fp.: 264°C (Zers.)

14 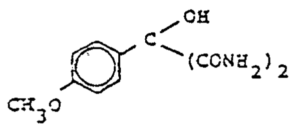 Fp.: 109 - 111°C (aus Chlorbenzol)

15 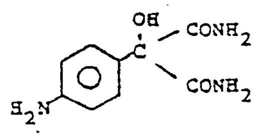 Fp.: 207 - 209°C (aus Ethanol)

16 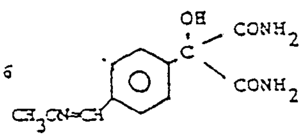 Fp.: 134 - 135°C (aus Ethanol)

17 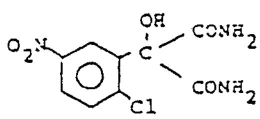 Fp.: 236 - 239°C (aus Butanol)

18 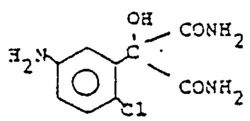 Fp.: 231 - 233°C

19 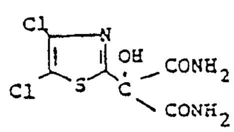 Fp.: 200 - 203°C (aus Isopropanol)

20 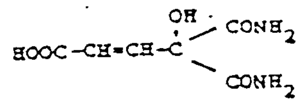 Fp. 160 - 162 (Z) (aus Acetonitril)

21 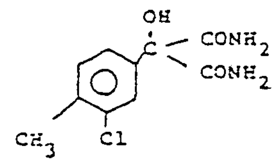 Fp. 178 - 179°C

Im folgenden werden weitere Beispiele zur Herstellung der Verbindungen der Formel I sowie zur Herstellung ihrer Ausgangsprodukte gegeben:

I) Herstellung der Verbindungen der Formel IX und ihre weitere Umsetzung zu Verbindungen der Formel III und ihre weitere Umsetzung zu Verbindungen der Formel I gemäß Verfahren b:

**Beispiel $Ia_1$**

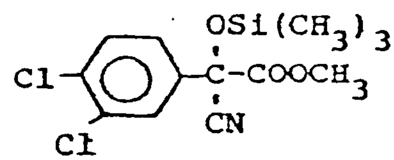

Zu 147,8 g 3,4-Dichlorphenylglyoxylsäuremethylester und 0,5 ml Triethylamin wurden unter Eiskühlung bei max. 55° C 62,8 g Trimethylsilylcyanid zugetropft. Es wurden 210 g 3,4-Dichlorphenyl-trimethylsilyloxy-malonsäuremethylesternitril erhalten. IR, NMR und MS bestätigen das Vorliegen der Substanz.

**Beispiel Ia$_2$**

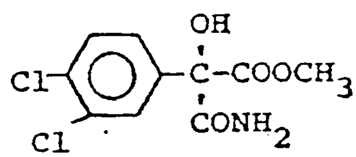

In 400 g Schwefelsäure (96 %ig) wurde bei max. 40° C 210 g 3,4-Dichlorphenyl-trimethylsilyloxy-malonsäureethylesternitril eingetropft. Es wurde noch 1 Stunde bei Raumtemperatur nachgerührt und dann in Eiswasser eingerührt.

Der zuerst schmierige, dann kristallisierende Niederschlag wurde in Essigsäureethylester aufgenommen, gewaschen, getrocknet und eingeengt.

Es wurden 130 g Rohprodukt erhalten.

Durch Umkristallisation aus Isopropanol wurde 3,4-Di-chlorphenyl-hydroxymalonsäuremethylesteramid vom Schmelzpunkt 132 - 133°C erhalten.

**Beispiel Ia$_3$**

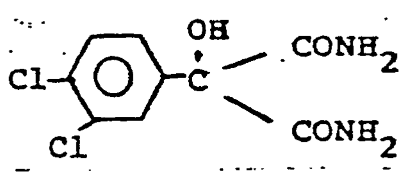

27,8 g 3,4-Dichlorphenylhydroxymalonsäuremethylesteramid wurden in 150 ml Methanol gelöst und Ammoniak eingeleitet. Die Reaktionstemperatur stieg dabei auf 40° C an.

Es wurde eingeengt und aus Isopropanol umkristallisert. Es blieben 22 g 3,4-Dichlorphenyl-hydroxymalonsäurediamid.

**Beispiel Ib$_1$**

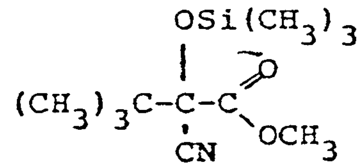

Analog zu Beispiel Ia$_1$ wurde aus 116,8 g -Keto-3,3-di-methylbuttersäuremethylester, 0,5 ml Triethylamin und 81 g Trimethylsilylcyanid 197,8 g tert.-Butyl-trimethylsilyloxy-malonsäuremethylesternitril hergestellt.

**Beispiel Ib$_2$**

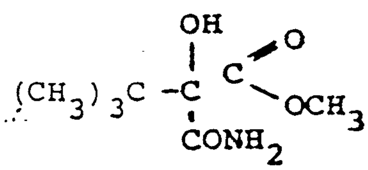

Analog zu Beispiel Ia$_2$ wurden aus 187 g tert.-Butyl-trimethylsilyloxy-malonsäuremethylesternitril durch Verseifung im 500 g $H_2SO_4$ 55 g tert.-Butylhydroxymalonaäuremethylesteramid hergestellt.

Schmelzpunkt 102 - 103°C (aus Waschbenzin).

II) Herstellung von Verbindungen der Formel IV und ihre weitere Umsetzung zu Verbindungen der Formel I ge-mäß Verfahren c):

**Beispiel IIb$_1$**

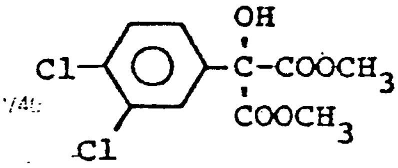

320 ml abs. Methanol wurden bei 10°C mit ca. 210 g Salzsäure gesättigt. Bei -10°C wurden 300 g

3,4-Dichlor-phenyl-trimethylsilyloxy-malonsäuredinitril zugetropft, 1 Stunde bei 10 - 15°C nachgerührt und bei 15°C mit 36 g Wasser verdünnt mit 50 ml Methanol, versetzt. Anschließend wurde 1 Stunde bei max. 30°C nachgerührt, von den ausgefallenen Kristallen abgesaugt und das Filtrat eingeengt. Es wurde mit Methylenchlorid aufgenommen, mit Eiswasser gewaschen, getrocknet, eingeengt und der Rückstand destilliert. Es wurden 165 g 3,4-Dichlorphenylhydroxy-malonsäuredimethylester erhalten. $Kp_{0,3}$ = 154 - 58°C.

**Beispiel IIb$_2$**

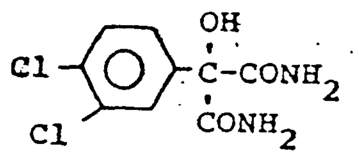

30 g 2,4-Dichlorphenyl-hydroxymalonsäuredimethylester wurden in 80 ml Methanol gelöst und $NH_3$ eingeleitet. Die Lösung erwärmte sich hierbei bis auf 50°C. Nach dem Einengen blieben 26 g 3,4-Dichlorphenyl-hydroxy-malonsäurediamid vom Fp = 175 - 77°C zurück.

**Beispiel A**

Phaedon-Larven-Test
Lösungsmittel: 3 Gewichtsteile
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik: 1, 3, 5, 6,

**Beispiel B**

Tetranychus-Test (resistent)
Lösungsmittel: 3 Gewichtsteile Dimethylformamid
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik. 5, 1, 3, 11, 6.

**Beispiel C**

Grenzkonzentrations-Test / Bodeninsekten
Testinsekt= Phaedon cochleariae-Larven (im Boden)
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der

Technik: 6, 7, 1, 3, 5.

**Patentansprüche**

1. Substituierte Hydroxymalonsäurediamide der Formel (I)

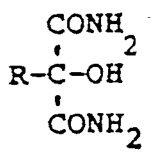

(I)

in welcher
R für Alkyl mit mindestens 2 C-Atomen sowie für durch Chlor substituiertes Alkyl, gegebenenfalls substituiertes Alkenyl, gegebenenfalls substituiertes Cycloalkyl, substituiertes Aryl, gegebenenfalls substituiertes Heteroaryl steht.

2. Verfahren zur Herstellung der substituierten Hydroxymalonsäurediamide der Formel I

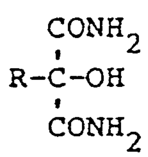

(I)

in welcher
R für Alkyl mit mindestens 2 C-Atomen sowie für durch Chlor substituiertes Alkyl, gegebenenfalls substituiertes Alkenyl, gegebenenfalls substituiertes Cycloalkyl, substituiertes Aryl, gegebenenfalls substituiertes Heteroaryl steht, dadurch gekennzeichnet, daß man

a) substituierte Trimethylsilyloxymalonsäuredinitrile der Formel (II)

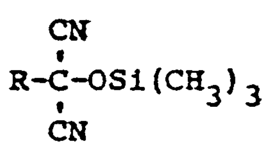

(II)

in welcher
R die oben angegebene Bedeutung hat,
mit anorganischen Säuren verseift, oder

b) Hydroxymalonsäureesteramide der Formel III

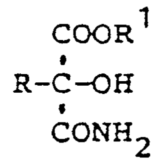

(III)

in welcher
R die oben angegebene Bedeutung hat und
$R^1$ für gegebenenfalls substituiertes Alkyl oder gegebenenfalls substituiertes Cycloalkyl steht
mit Ammoniak umsetzt
oder

c) Hydroxylmalonsäureester der Formel IV

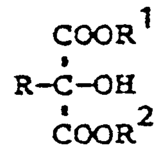

(IV)

in welcher
R bzw. $R^1$ die oben angegebene Bedeutung haben und
$R^2$ die für $R^1$ angegebene Bedeutung hat, wobei $R^1$ und $R^2$ gleich oder verschieden sein können
mit Ammoniak umsetzt
oder

d) Hydroxy- bzw. Trimethylsilyloxymalonsäureamidnitrile der allgemeinen Formel V

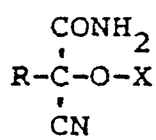

(VI)

in welcher
R die oben angegebene Bedeutung hat und
X für Wasserstoff oder $Si(CH_3)_3$ steht
mit anorganischen Säuren verseift
oder

e) Acyloxymalonsäurediamide der allgemeinen Formel

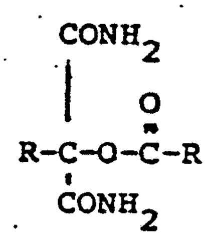

(VI)

in welcher
R die oben angegebene Bedeutung hat
in Gegenwart von Basen verseift
oder

f) Acyloxymalonsäuredinitrile der allgemeinen Formel VII

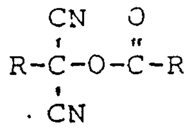

VII

in welcher

R die oben angegebene Bedeutung hat mit anorganischen Mineralsäuren verseift

oder

g) Acyloxymalonsäurediester der allgemeinen Formel VIII

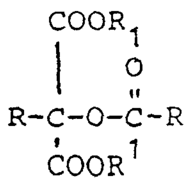

(VIII)

in welcher

R und $R^1$ die oben angegebene Bedeutung haben mit Ammoniak umsetzt.

3. Verbindungen der Formel I gemäß Anspruch 1, in welcher

R für $C_{2-4}$-Alkyl, für durch Chlor substituiertes $C_{1-4}$-Alkyl, für $C_{2-7}$-Alkenyl, $C_{3-6}$-Cycloalkyl steht oder für $C_{2-7}$-Alkenyl, $C_{3-6}$-Cycloalkyl steht, die gleich oder verschieden durch einen oder mehrere der folgenden Reste substituiert sind:

Halogen, $C_{1-4}$-Alkoxy, Carboxyl, Carbalkoxy, Phenyl, Phenoxy, Thiophenyl wobei die Phenylringe durch Halogen oder Alkyl substituiert sein können;

oder in welcher R ferner für Phenyl steht, das gleich oder verschieden durch einen oder mehrere der folgenden Reste substituiert ist: Halogen, Nitro, Amino, CN, OH, $C_{1-4}$-Alkyl, $C_{1-4}$-Halogenalkyl, $C_{1-4}$-Alkoxy, $C_{1-4}$-Halogenalkoxy, Methylendioxy, Ethylendioxy, Difluormethylendioxy, halogensubstituiertes Ethylendioxy, $C_{1-4}$-Alkylthio, $C_{1-4}$-Halogenalkylthio, $C_{2-8}$-Alkoxyalkyl, $C_{2-8}$-Halogenalkoxyalkyl, $C_{1-4}$-Alkylsulfonyl, $C_{1-4}$-Halogenalkylsulfonyl, Carboxyl, Carbalkoxy

sowie den Rest $C_{1-4}$-Alkoxy-N=CH-, ferner für Phenyl, Phenyloxy, Thiophenyl die gegebenenfalls durch Halogen oder $C_{1-4}$-Alkyl substituiert sein können, sowie für Carbalkoxy mit 2 - 4 C-Atomen,

oder wobei R ferner für Pyridinyl, Pyrimidinyl, Triazinyl, Isoxazolyl, Thiazolyl, Oxadiazolyl, Imidazolyl, Triazolyl, Furanyl, Thiophenyl steht, die gegebenenfalls ein oder mehrfach gleich oder verschieden durch Halogen, $C_{1-4}$-Alkyl oder $C_{1-4}$-Alkoxy substituiert sein können.

4. Verbindungen der Formel I gemäß Anspruch 1

in welcher

R für $C_{2-4}$-Alkyl, für chlorsubstituiertes $C_{1-4}$-Alkyl sowie für durch Halogen, Phenyl oder Phenoxy substituiertes $C_{5-6}$-Cycloalkyl, für gegebenenfalls durch Carboxyl substituiertes $C_{2-4}$-Alkenyl, ferner für Phenyl, das durch Halogen, $C_{1-4}$-Alkyl, $C_{1-4}$-Alkoxy, $C_{1-4}$-Halogen-alkoxy, $C_{1-4}$-Halogenalkyl, $NH_2$, $CH_3O_2$-N=CH-, oder Nitro substituiert ist, steht.

5. Verbindungen in welcher

R für Phenyl steht, das ein- oder mehrfach durch Chlor oder Fluor substituiert ist.

6. Schädlingsbekämpfungsmittel, gekennzeichnet durch einen Gehalt an mindestens einem substituierten Hydroxymalonsäurediamid der Formel I gemäss Anspruch 1.

7. Verwendung von substituierten Hydroxymalonsäurediamiden der Formel Ia

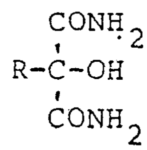

(Ia)

in welcher

R für Alkyl mit mindestens 2 C-Atomen sowie für substituiertes Alkyl, gegebenenfalls substituiertes Alkenyl, gegebenenfalls substituiertes Cycloalkyl, gegebenenfalls substituiertes Aryl, gegebenenfalls substituiertes Heteroaryl steht, zur Bekämpfung von Schädlingen.

8. Verfahren zur Bekämpfung von Schädlingen, dadurch gekennzeichnet, daß man substituierte Hydroxymalonsäurediamide der Formel (Ia) gemäß Anspruch 7 auf Schädlinge und/oder ihren Lebensraum einwirken läßt.

9. Verfahren zur Herstellung von Schädlingsbekämpfungsmitteln, dadurch gekennzeichnet, daß man substituiertes Hydroxymalonsäurediamid der Formel (Ia) gemäß Anspruch 7 mit Streckmitteln und/oder oberflächenaktiven Mitteln vermischt.

## Claims

1. Substituted hydroxymalonic acid diamides of the formula (I)

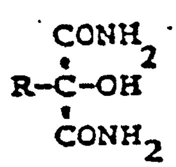

(I)

in which

R represents alkyl with at least 2 C atoms and chlorinesubstituted alkyl, optionally substituted alkenyl, optionally substituted cycloalkyl,

substituted aryl, or optionally substituted heteroaryl.

2. Process for the preparation of the substituted hydroxymalonic acid diamides of the formula I

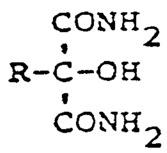

(I)

in which

R represents alkyl with at least 2 C atoms and chlorinesubstituted alkyl, optionally substituted alkenyl, optionally substituted cycloalkyl, substituted aryl, or optionally substituted heteroaryl,

characterised in that

a) substituted trimethylsilyloxymalonic acid dinitriles of the formula (II)

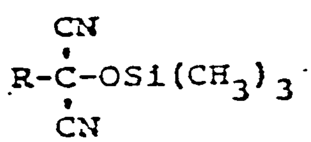

(II)

in which

R has the abovementioned meaning,

are saponified with inorganic acids or

b) hydroxymalonic acid ester amides of the formula III

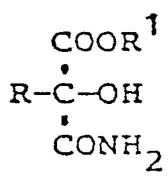

(III)

in which

R has the abovementioned meaning and

$R^1$ represents optionally substituted alkyl or optionally substituted cycloalkyl,

are reacted with ammonia or

c) hydroxymalonic acid esters of the formula IV

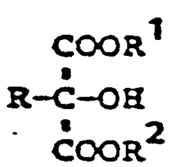

(IV)

in which

R and $R^1$ have the abovementioned meaning and

$R^2$ has the meaning given for $R^1$, it being possible for

$R^1$ and $R^2$ to be identical or different,

are reacted with ammonia or

d) hydroxy- or trimethylsilyloxy-malonic acid amide nitriles of the general formula V

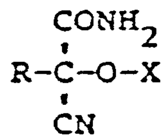

(VI)

in which

R has the abovementioned meaning and

X represents hydrogen or $Si(CH_3)_3$,

are saponified with inorganic acids or

e) acyloxymalonic acid diamides of the general formula

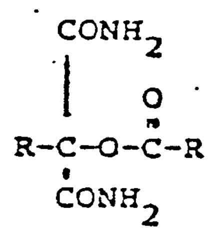

(VI)

in which

R has the abovementioned meaning,

are saponified in the presence of bases or

f) acyloxymalonic acid dinitriles of the general formula VII

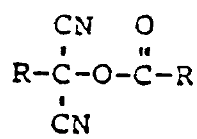

VII

in which

R has the abovementioned meaning

are saponified with inorganic mineral acids or

g) acyloxymalonic acid diesters of the general formula VIII

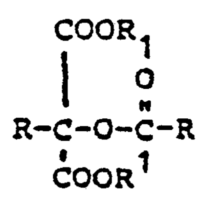

(VIII)

in which

R and $R^1$ have the abovementioned meaning,

are reacted with ammonia.

3. Compounds of the formula 1 according to Claim 1, in which R represents $C_{2-4}$-alkyl, chlorine-substituted $C_{1-4}$-alkyl, $C_{2-7}$-alkenyl or $C_{3-6}$-cycloalkyl, or represents $C_{2-7}$-alkenyl, or $C_{3-6}$-

cycloalkyl, which are substituted by one or more of the following radicals, the substituents being identical or different:

halogen, $C_{1-4}$-alkoxy, carboxyl, carbalkoxy, phenyl, phenoxy, or thiophenyl, it being possible for the phenyl rings to be substituted by halogen or alkyl;

or in which

R furthermore represents phenyl which is substituted by one or more of the following radicals, the substituents being identical or different:

halogen, nitro, amino, CN, CH, $C_{1-4}$-alkyl, $C_{1-4}$-halogenoalkyl, $C_{1-4}$-alkoxy, $C_{1-4}$-halogenoalkoxy, methylenedioxy, ethylenedioxy, difluoromethylenedioxy, halogen-substituted ethylenedioxy, $C_{1-4}$-alkylthio, $C_{1-4}$-halogenoalkylthio, $C_{2-8}$-alkoxyalkyl, $C_{2-8}$-halogenoalkoxyalkyl, $C_{1-4}$-alkyl-sulphonyl, $C_{1-4}$-halogenoalkylsulphonyl, carboxyl, or carbalkoxy,

and the radical $C_{1-4}$-alkoxy-N=C-, also phenyl, phenyloxy, thiophenyl, which can optionally be substituted by halogen or $C_{1-4}$-alkyl, and carbalkoxy with 2-4 C atoms, or wherein

R furthermore represents pyridinyl, pyrimidinyl, triazinyl, isoxazolyl, thiazolyl, oxadiazolyl, imidazolyl, triazolyl, furanyl or thiophenyl, which can optionally be substituted by one or more identical or different substituents, these being halogen, $C_{1-4}$-alkyl or $C_{1-4}$-alkoxy.

4. Compounds of the formula I according to Claim 1,

in which

R represents $C_{2-4}$-alkyl, chlorine-substituted $C_{1-4}$-alkyl and halogen-, phenyl- or phenoxy-substituted $C_{5-6}$-cycloalkyl, optionally carboxyl-substituted $C_{2-4}$-alkenyl, also phenyl which is substituted by halogen, $C_{1-4}$-alkyl, $C_{1-4}$-alkoxy, $C_{1-4}$-halogenoalkoxy, $C_{1-4}$-halogenoalkyl, $NH_2$, $CH_3O$-N=CH-, or nitro.

5. Compounds in which

R represents phenyl which is mono- or polysubstituted by chlorine or fluorine.

6. Agents for combating pests, characterised in that they contain at least one substituted hydroxymalonic acid diamide of the formula I according to Claim 1.

7. Use of substituted hydroxymalonic acid diamides of the formula Ia

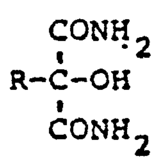

(Ia)

in which

R represents alkyl with at least 2 C atoms and substituted alkyl, optionally substituted alkenyl, optionally substituted cycloalkyl, optionally substituted aryl or optionally substituted heteroaryl,

for combating pests.

8. Method of combating pests, characterised in that substituted hydroxymalonic acid diamides of the formula (Ia) according to Claim 7 are allowed to act on pests and/or their habitat.

9. Process for the preparation of agents for combating pests, characterised in that substituted hydroxymalonic acid diamide of the formula (Ia) according to Claim 7 is mixed with extenders and/or surface-active agents.

**Revendications**

1. Diamides substitués de l'acide hydroxymalonique de formule (I):

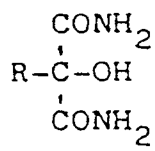

(I)

dans laquelle

R représente un groupe alkyle contenant au moins 2 atomes de carbone, ainsi qu'un groupe alkyle substitué par le chlore, un groupe alcényle éventuellement substitué, un groupe cycloalkyle éventuellement substitué, un groupe aryle substitué ou un groupe hétéro-aryle éventuellement substitue.

2. Procédé de préparation des diamides substitués de l'acide hydroxy-malonique de formule I:

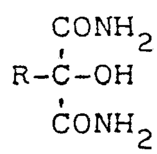

(I)

dans laquelle

R représente un groupe alkyle contenant au moins 2 atomes de carbone, ainsi qu'un groupe alkyle substitué par le chlore, un groupe alcényle éventuellement substitué, un groupe cycloalkyle éventuellement substitué, un groupe aryle substitué ou un groupe hétéro-aryle éventuellement substitue, caractérisé en ce que:

a) on saponifie des dinitriles substitués de l'acide triméthylsilyloxy-malonique de formule (II):

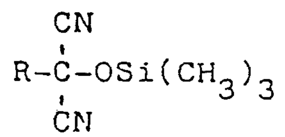

(II)

dans laquelle

R a la signification indiquée ci-dessus, avec des acides inorganiques, ou

b) on fait réagir des esteramides de l'acide hydroxy-malonique de formule III:

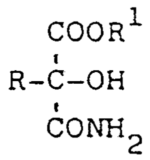

(III)

dans laquelle
R a la signification indiquée ci-dessus, et
$R^1$ représente un groupe alkyle éventuellement substitué ou un groupe cycloalkyle éventuellement substitué,
avec l'ammoniac,
ou
c) on fait réagir des esters de l'acide hydroxymalonique de formule IV:

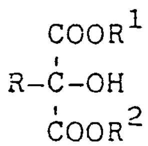

(IV)

dans laquelle
R et $R^1$ ont les significations indiquées ci-dessus, et $R^2$ a la signification indiquée pour $R^1$, $R^1$ et $R^2$ pouvant être identiques ou différents,
avec l'ammoniac,
ou
d) on saponifie des amido-nitriles de l'acide hydroxy- ou triméthylsilyloxy-malonique de formule générale V:

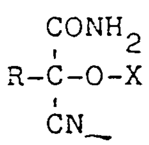

(V)

dans laquelle
R a la signification indiquée ci-dessus, et
X représente l'hydrogéne ou $Si(CH_3)_3$,
avec des acides inorganiques,
ou
e) on saponifie des diamides d'acides acyloxymaloniques de formule générale:

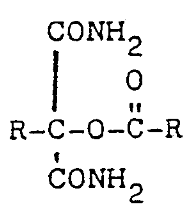

(VI)

dans laquelle
R a la signification indiquée ci-dessus,
en présence de bases,
ou
f) on saponifie des dinitriles d'acides acyloxymaloniques de formule générale VII:

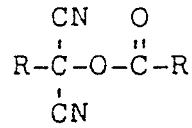

(VII)

dans laquelle
R a la signification indiquée ci-dessus,
avec des acides minéraux inorganiques,
ou
g) on fait réagir des diesters d'acides acyloxymaloniques de formule générale VIII

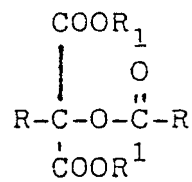

(VIII)

dans laquelle
R et $R^1$ ont les significations indiquées ci-dessus, avec l'ammoniac.

3. Composés de formule I selon la revendication 1, formule dans laquelle
R représente un groupe alkyle en $C_2$-$C_4$, un groupe alkyle en $C_1$-$C_4$ substitué par le chlore, un groupe alcényle en $C_2$-$C_7$, un groupe cycloalkyle en $C_3$-$C_6$, un groupe alcényle en $C_2$-$C_7$ ou un groupe cycloalkyle en $C_3$-$C_6$, ces groupes étant substitués de manière identique ou différente par un ou plusieurs des radicaux suivants:
les atomes d'halogénes, les groupes alcoxy en $C_1$-$C_4$, carboxy, carbalcoxy, phényle, phénoxy et thiophényle, les noyaux phényle pouvant étre substitués par un atome d'halogène ou par un groupe alkyle;
ou dans laquelle R représente, en outre, un groupe phényle qui est substitué de manière identique ou différente par un ou plusieurs des radicaux suivants: les atomes d'halogènes, les groupes nitro, amino, CN, OH, alkyle en $C_1$-$C_4$, halogénalkyle en $C_1$-$C_4$, alcoxy en $C_1$-$C_4$, halogénalcoxy en $C_1$-$C_4$, méthyléne-dioxy, éthylène-dioxy, difluorométhylenedioxy, éthylène-dioxy substitué par un atome d'halogène, alkyl(en $C_1$-$C_4$)thio, halogénalkyl(en $C_1$-$C_4$) thio, alcoxy-alkyle en $C_2$-$C_8$, halogenalcoxy-alkyle en $C_2$-$C_8$, alkyl(en $C_1$-$C_4$)sulfonyle, halogénalkyl(en $C_1$-$C_4$)sulfonyle, carboxy, carbalcoxy, ainsi que le radical alcoxy(en $C_1$-$C_4$)-N=CH- et les groupes phényle, phényloxy et thiophényle qui peuvent éventuellement être substitués par un atome d'halogène ou par un groupe alkyle en $C_1$-$C_4$, de même que les groupes carbalcoxy contenant 2 à 4 atomes de carbone,
ou R représente, en outre, un groupe pyridinyle, un groupe pyrimidinyle, un groupe triazinyle, un groupe isoxazolyle, un groupe thiazolyle, un groupe oxadiazolyle, un groupe imidazolyle, un groupe triazolyle, un groupe furannyle, ou un groupe thiophényle, ces groupes pouvant être éventuellement substitués une ou plusieurs fois

de manière identique ou différente par un atome d'halogène, par un groupe alkyle en $C_1$-$C_4$ ou par un groupe alcoxy en $C_1$-$C_4$.

4. Composés de formule I selon la revendication 1, formule dans laquelle

R représente un groupe alkyle en $C_2$-$C_4$, un groupe alkyle en $C_1$-$C_4$ substitué par le chlore, ainsi qu'un groupe cycloalkyle en $C_5$-$C_6$ substitué par un atome d'halogène, par le groupe phényle ou par le groupe phénoxy, un groupe alcényle en $C_2$-$C_4$ éventuellement substitué par un groupe carboxy, de même qu'un groupe phényle substitué par un atome d'halogène, par un groupe alkyle en $C_1$-$C_4$, par un groupe alcoxy en $C_1$-$C_4$, par un groupe halogénalcoxy en $C_1$-$C_4$, par un groupe halogénalkyle en $C_1$-$C_4$, par $NH_2$, par $CH_3O$-N=CH- ou par le groupe nitro.

5. Composés dans lesquels

R représente un groupe phényle substitue une ou plusieurs fois par le chlore ou le fluor.

6. Parasiticides, caractérisés en ce qu'ils contiennent au moins un diamide substitué d'acide hydroxy-malonique de formule I selon la revendication 1.

7. Utilisation de diamides substitués de l'acide hydroxy-malonique de formule Ia:

FIG71/30

dans laquelle

R représente un groupe alkyle contenant au moins 2 atomes de carbone, ainsi qu'un groupe alkyle substitué, un groupe alcényle éventuellement substitué, un groupe cycloalkyle éventuellement substitué, un groupe aryle éventuellement substitué ou un groupe hétéro-aryle éventuellement substitué, pour combattre les parasites.

8. Procédé en vue de combattre les parasites, caractérisé en ce qu'on fait agir des diamides substitués de l'acide hydroxy-malonique de formule (Ia) selon la revendication 7 sur les parasites et/ou leur biotope.

9. Procédé de préparation de parasiticides, caractérisé en ce qu'on mélange un diamide substitué de l'acide hydroxy-malonique de formule (Ia) selon la revendication 7 avec des diluants et/ou des agents tensio-actifs.